Europäisches Patentamt

**European Patent Office**

Office européen des brevets

(19)

(11) Publication number: **0 147 234**

**A2**

(12) **EUROPEAN PATENT APPLICATION**

(21) Application number: **84309118.2**

(22) Date of filing: **28.12.84**

(51) Int. Cl.⁴: **C 07 B 61/00**
**C 07 C 1/00**

(30) Priority: **29.12.83 US 566869**

(43) Date of publication of application:
**03.07.85 Bulletin 85/27**

(84) Designated Contracting States:
**BE DE FR GB IT NL**

(71) Applicant: **E.I. DU PONT DE NEMOURS AND COMPANY**
**1007 Market Street**
**Wilmington Delaware 19898(US)**

(72) Inventor: **Santorsa, Michael Paul**
**9 Edgewood Drive R.F.D. 3**
**Hudson New Hampshire 03051(US)**

(72) Inventor: **Urry, Grant Wayne**
**2 Blackhorse Terrace**
**Winchester Massachusetts 01890(US)**

(74) Representative: **Jones, Alan John et al,**
**CARPMAELS & RANSFORD 43 Bloomsbury Square**
**London, WC1A 2RA(GB)**

(54) Synthesis of organic compounds from carbon.

(57) A relatively low-temperature and low-pressure synthesis of hydrocarbons, carboxylic acids, alcohols, and nitrogen- or sulfur-containing organic compounds. The method comprises reacting a carbon-source with aqueous base at reaction temperatures of about 200°C to 450°C.

EP 0 147 234 A2

Croydon Printing Company Ltd.

## TITLE

## SYNTHESIS OF ORGANIC
## COMPOUNDS FROM CARBON

### BACKGROUND OF THE INVENTION

This invention comprises a method for making organic compounds of varying carbon length and geometry under relatively mild conditions from carbon, water, and base. The method is flexible in that nitrogen and/or sulfur can be employed along with the carbon, water and base to extend the range of products that are produced.

Representative prior art methods that employ carbon or a carbon-source to make hydrocarbon products usually employ catalysts, superatmospheric pressures and/or high temperatures. Such processes produce significant amounts of gaseous products such as methane, carbon dioxide, carbon monoxide and/or hydrogen. Representative of such art are the following publications.

Cabrera et al., "Methane Production from the Catalyzed Reaction of Graphite and Water Vapor at Low Temperatures (500-600K)", J. Catalysis 75, pages 7 to 22 (1982) disclose coating a graphite surface with KOH, $K_2CO_3$, LiOH or NaOH and heating to reaction temperature at pressures up to 100 atm. Products were $CH_4$ and $CO_2$ or $CH_4$ and CO.

Guzman et al., "Kinetics of the $K_2CO_3$ – Catalyzed Steam Gasification of Carbon and Coal", Ind. Eng. Chem. Process Des. Dev. 21, pages 25 to 29 (1982) disclose the atmospheric pressure kinetics of the $K_2CO_3$-catalyzed steam gasification of activated carbon and coal char. The main reaction products were $CO_2$, CO and $H_2$.

8182

Nahas et al., "Catalytic Gasification Predevelopment Research", Proceedings of the Thirteenth Intersociety Energy Conversion Engineering Conference, pages 2143 to 2147 (Aug. 20 to 25, 1978) disclose that potassium is an active methanation catalyst lowering the gasification temperature from 925°C to 700°C.

Woodcock, "Catalytic Coal Gasification Exploratory Research Program", Proceedings of the Thirteenth Intersociety Energy Conversion Engineering Conference, pages 2148 to 2152 (Aug. 20 to 25, 1978) discloses a process employing additives to "catalyze" coal gasification reactions. The additives include hydroxides and carbonates of K, Na, and Li.

Ostermann et al., "Kinetics of the Thermochemical Conversion of Cellulose to Oil in Aqueous Alkaline Solution", Energy From Biomass and Wastes IV, Symposium (1980) pages 645 to 658 disclose the conversion of cellulose to oil by heating in aqueous sodium carbonate solution at about 250° to 310°C with or without added carbon monoxide. The reference discloses the use of up to 0.2 g of sodium carbonate per g of cellulose, which would give a $Na^+$/C molar ratio of 0.1. The disclosure refers to tar and synthetic coal which are not products of the instant invention.

Other publications include the following: (1) Heinemann, Petroleum Refiner 29, 111 to 114 (1950). (2) Heinemann, Petroleum Refiner 33, 161 to 163 (1954). (3) Casanova et al., Fuel 62, 1138 to 1144 (1983). (4) U.S. Patents 2,461,740; 4,113,446; 4,345,098; 2,193,337; 2,786,074; 4,094,650. (5) Ross et al., Fluid Phase Equilibria 10, pages 319 to 326 (1983). This reference teaches coal conversion to hydrogen-enriched, benzene-soluble products in aqueous systems, adjusted to an initial pH of 12.6. Base is employed in catalytic quantities, and the molar ratios of metal ions to carbon and water are relatively low. (6) Heinemann et al., "Chemistry and Morphology of Coal Liquification". Department of Energy Heterogeneous Catalysis Meeting. November 7 to 9, 1983, NBS, Gaithersburg, Maryland, in an oral presentation disclosed a reaction of carbon and water in the presence of alkali hydroxide to produce methane and carbon dioxide at 225°C. At 500°C, hydrocarbons from $C_1$ to $C_6$, mostly olefinic from $C_2$ to $C_6$ were produced. (7) Nekleevich et al., Chem. Abs. $\underline{38}$, 6614 (1938), disclose heating a mixture of 50 g of soot carbon, 200 g of sodium hydroxide and 180 mL of water to 380°C and 43 atm pressure to give hydrocarbons. Oxygenated organic products are not disclosed. Calculated molar ratios: $Na^+/C = 1.2$; $Na^+/H_2O = 0.5$; and $C/H_2O = 0.42$.

## SUMMARY OF THE INVENTION

This invention comprises a method for making organic compounds which contain carbon, hydrogen, and at least one element selected from the group consisting of oxygen, nitrogen, and sulfur, characterized by contacting these reactants:

        (i) a carbon-source,

        (ii) water,

        (iii) base, and, optionally,

        (iv) a heteroatom-source,

at temperatures of about 200°C to 450°C, the method characterized further in that:

        (v) the base is selected from alkali and alkaline earth hydroxides and carbonates having a cation, $M^{+n}$, wherein n is 1 or 2,

(vi)     the molar ratio of $M^{+n}$/C is at least about 0.06/n,

(vii)    the molar ratio of $M^{+n}$/water is at least about 0.05/n, and

(viii)   the molar ratio of carbon in the carbon-source/water is at least about 0.2.

For best results, preferred temperatures are 350°C to 400°C. Preferred bases are potassium hydroxide, sodium hydroxide, calcium hydroxide, magnesium hydroxide, potassium carbonate, sodium carbonate, calcium carbonate and magnesium carbonate, lithium hydroxide and lithium carbonate. The most preferred bases are sodium hydroxide, calcium hydroxide, and potassium hydroxide. The preferred carbon sources are coals, lignites, and carbohydrates. Heteroatom-sources include nitrogen gas, $Al_2O_3$, $B_2O_3$, alkali metal sulfates and hydrogen sulfide. The preferred heteroatom-sources are nitrogen gas, $Al_2O_3$ and $B_2O_3$.

At the preferred temperatures of 350° to 400°C, the reaction contact time may be only a few minutes for a finely divided carbon-source. At temperatures of 200° to 250°C, several hours may be required. By "heteroatom-source" is meant any compatible reactant that provides elements other than carbon, hydrogen and oxygen for incorporation into one or more of the final products.

The molar ratio characterizations (vi), (vii) and (viii) have been found to be important to the method of this invention. For best results, it has been found that all three of the ratios should meet or exceed the disclosed minimum values. It has also been found, however, that acceptable results are sometimes achieved when one of the three ratios is slightly below its disclosed minimum value. Accordingly, it is contemplated that the method of this invention includes reactions in which one of ratios (vi), (vii) or (viii) falls below its recommended minimum value by no more than about 20%.

The contemplated molar ratio (vi) of $M^{+n}/C$ is between about 0.06/n to 2/n. The preferred ratio is about 0.3/n to 1/n. The molar ratio (vii) of $M^{+n}$/water is between about 0.05/n to 2/n. The preferred ratio is about 0.1/n to 1/n. The molar ratio (viii) of carbon/water is between about 0.2 to 5. The preferred ratio is about 0.3 to 1.

Batch, semibatch and continuous processes can be employed and both contact time and pressure will vary considerably. Autogenous pressure is preferred in batch operation, and pressures corresponding to autogenous are preferred for continuous reaction. Slurry reactors that provide suitable contact among solid particles, liquid, and gas can be used to operate the method of this invention continuously. The operating conditions and feed ratios used are preferably those specified for a batch reactor.

## DETAILS OF THE INVENTION

### Carbon-Source

Virtually any carbon-source except diamond can be employed in which the oxidation state of the carbon is lower than that in carbon dioxide. As a practical matter, compounds containing about 40% or more of carbon are preferred. When the carbon-source contains bound hydrogen and oxygen, production is facilitated of products such as $C_1$ to $C_7$ alkanes, $C_2$ to $C_7$ alkenes, $C_5$ to $C_7$ dienes, benzene, toluene, xylene, phenol, cresol, and the like. Contemplated carbon-sources include coals such as anthracite, bituminous coal, subbituminous coal, lignite, humic acid, peat, and pitch; carbohydrates such as cellulose, starch, and sugar; biomass such as wood chips, corncobs, plant wastes, leaves, potatoes, grass, garbage, sewage sludge, and manure; petroleum and petroleum residue, char, tar, coke, shale; poly(tetrafluoroethylene) and other halogenated hydrocarbons; methane and carbon monoxide.

Water and Base

The base is not employed in catalytic quantities. The base can be employed singly or mixtures of bases can be used. It is a reactant needed in stoichiometric quantities. Sufficient excess base is usually employed to give a solution pH of about 8 to 14 at the conclusion of the reaction. The overall general reaction can be viewed as a $C/H_2O$ redox system. Part of the carbon is oxidized to a carbonate or bicarbonate and the rest of the carbon is reduced to organic materials. Water provides both the hydrogen for the reduction of the carbon and the oxygen for the oxidation of the carbon. The ratios of water and base relative to each other and to carbon have been described in the "Summary" as have the particular bases contemplated for use.

Heteroatom-Source

In addition to water, contemplated sources of compound constituents other than carbon, hydrogen, and oxygen include: nitrogen gas, nitrogen-containing compounds which give up nitrogen under reaction conditions; $Al_2O_3$, $B_2O_3$, alkali metal sulfates and hydrogen sulfide. When ammonia is the desired product, a source of nitrogen, preferably nitrogen gas, is employed as the heteroatom-source.

Ammonia, pyridine and amino acids are produced when nitrogen gas is employed as a heteroatom-source. Nitrogen gas pressures above about 1 atmosphere (0.1 MPa) can be employed but pressures above about 35 atmospheres (3.5 MPa) are preferred. Under such conditions, nitrogen is incorporated into some of the products and extensive modification of the basic reaction occurs. Products include ammonia, pyridine, ethane, propylene, benzene and toluene.

Additives

To further promote selectivity of product formation, it may be desirable to add a selected additive to the reaction mixture. For instance, Example 28 shows that addition of manganese dioxide promotes the formation of alcohol products. Additives can be added in catalytic quantities, or amounts up to about stoichiometric amounts of the carbon-source. Suitable additives include manganese dioxide, molybdenum sources such as molybdenum oxides; oxides and carbonyls of iron, chromium, rhenium, nickel and other transition metals; phosphorus oxides; zinc oxide; sources of germanium, e.g., germanium oxide; tin oxide; antimony oxide; silicon dioxide; and hydrogen gas. A preferred additive is manganese dioxide.

It is characteristic of the method of this invention that the nature of the products can be readily altered by varying the reaction mix. This tunability is evident, for example, with regard to the carboxylates that are produced. When graphite was used as a carbon source, strong potassium hydroxide solutions gave largely carboxylates. With weaker solutions of potassium hydroxide or with metal carbonates, $C_{12}$ to $C_{17}$ alkanes were found

together with smaller quantities of carboxylates.
When coal was used as a carbon source, principal
components of the product mix included $C_1$ to $C_7$
alkanes, $C_2$ to $C_7$ alkenes, and $C_5$ to $C_7$
dienes.

When a metal oxide heteroatom-source such as
$B_2O_3$ or $Al_2O_3$, or a metal oxide additive such
as $MnO_2$ is employed, alcoholates and
tetrahydrofuran are produced in place of
carboxylates. The molar ratio of metal oxide to
carbon should be at least about 0.05 with 0.2 to 0.3
being preferred. The alcoholates produced include
those of methanol, isopropyl alcohol, tert-butyl
alcohol, and pentanol. The metal oxide additions
also reduce the number of aromatic materials normally
produced. When $B_2O_3$ and $Al_2O_3$ were added to
runs using mineral refining wastes as a carbonaceous
source, the effect of the metal oxides was
moderated. Perhaps because of the relatively high
mineral content, fewer carboxylic acids were
produced, no alcohols were produced and
tetrahydrofuran was found. The alkanes and alkenes
normally found with carbonaceous sources containing
bound hydrogen and oxygen were fewer in number and
the $C_5$ to $C_7$ dienes were replaced by cyclic $C_5$
and $C_6$ compounds.

### EXAMPLES

Listed in the following Examples, under the
column heading "Product Analyses", are the products
actually detected. Analytical detection of these
products may have been by gas phase and/or liquid
phase analyses including GC/MS. The fact that a
particular product analysis is not provided in a
specific instance does not necessarily mean that none
of that product was present. Rather, the lack of such analysis should also be understood to include the alternative explanation that no analysis was undertaken for that product. In these Examples, emphasis is placed on products actually analyzed. Calculated reactant ratios appear in Table 6 for each Example.

Apparatus/Procedures

All of the Examples were carried out in 10 mL Autoclave Engineers shaker bombs which were rapidly heated or cooled by immersing the tubes in or withdrawing them from a sandbath at reaction temperature. The Hastelloy C bombs contained a 5/16-inch (0.8-cm) diameter Hastelloy C ball, and they were oscillated vertically at 327 cycles per minute while in the sand bath. Heat-up required about 5 minutes and cooling occurred in 15 minutes. Unless otherwise indicated, runs were at 380°C for 8 hours. Pressure measurements are included in the Tables where available.

The general procedure for each run consisted of loading a shaker bomb in a dry box with a designated base and carbon source. When potassium hydroxide and graphite were employed, Fisher Scientific Company certified ACS grade potassium hydroxide containing about 15% water, and Fisher Scientific Company ACS grade powdered graphite were charged. Subsequently, distilled, deionized water was added, and after sealing, the bomb was cooled to −78°C and evacuated.

At the end of a run, the bombs were usually found to be under vacuum. When pressure was found, the amount of gas was measured. The gas samples were analyzed by a GC/MS system consisting of a Varian Model 3700 gas chromatograph and a V. G. Micromass Ltd. Model MM16F mass spectrometer.

Liquid and solid contents of the shaker bomb were removed and acidified with strong mineral acid to a pH of one. The acidified material was filtered to remove unreacted carbonaceous feed material and the precipitate was thoroughly washed with water. The aqueous solution was extracted with methyl tert-butyl ether, and the extract was concentrated by passing a stream of dry nitrogen over the surface of the liquid. The residue was then analyzed by GC/MS. Deviations from this procedure are noted for the specific runs involved.

In the summary Tables, aromatic compounds have the following codes:

| Aromatic Compound | Code |
|---|---|
| Benzene | B |
| Toluene | T |
| Xylene | X |
| Phenol | P |
| Cresol | C |
| Di-tert-butyl cresol | D |
| Dibutyl phthalate | I |

In the summary Tables, carbonaceous materials have the following codes:

| | | Ultimate Analysis (%) | | | |
|---|---|---|---|---|---|
| Code | Description | C | H | O | Other |
| G | Graphite | 99 | – | – | 1 |
| ANT | Anthracite | -- | – | – | – |
| CWK | Cimarron West Kentucky 11 Coal | 72 | 5 | 8 | 15 |
| ATL | Alcoa Texas Lignite | 57 | 4 | 16 | 23 |
| WSB | Wyodak Subbituminous Coal | 68 | 5 | 18 | 9 |
| MRW | Mineral Refining Waste | 55 | 4 | 39 | 2 |
| DEX | Dextrose | 41 | 7 | 52 | 0 |

## EXAMPLES 1 TO 15

These Examples illustrate the general reaction for the conversion of carbonaceous material to useful compounds. The results set forth in Tables 1 to 3 were obtained in the apparatus previously described. The feed and product analyses are indicated by appropriate Table headings. The bases employed in the Examples in Tables 1 and 2 are hydroxides. Those used in the Examples summarized in Table 3 are carbonates.

In all Examples in which it is employed, the amount of potassium hydroxide designated is the amount of 85% potassium hydroxide added as described above. The molar ratios shown in Table 6, when potassium hydroxide was the base, were calculated by including the water in the potassium hydroxide as part of the total moles of water charged. Alkane and carboxylic acid products include straight- and branched-chain species.

## TABLE 1

| | H$_2$O Added | Carbon | | Base | | Product Analysis | |
|---|---|---|---|---|---|---|---|
| Example | g | Type | g | Type | g | Carboxylic Acid, C$_n$ | Aromatic |
| 1 | 1.00 | G | 0.52 | KOH | 2.24 | 2 to 6 | PDI |
| 2 | 2.00 | G | 0.52 | KOH | 2.24 | 2 to 6 | PDI |
| 3 | 4.00 | G | 0.52 | KOH | 2.24 | 2 to 7 | PDI |
| 4[a] | 2.13 | G | 1.04 | KOH | 4.47 | 2 to 6 | PD |
| 5 | 1.00 | G | 0.52 | KOH | 1.00 | 2 to 7 | D |
| 6 | 1.00 | G | 0.52 | KOH | 0.17 | 2 to 7 | D |
| 7 | 1.00 | G | 0.52 | Ca(OH)$_2$ | 2.28 | 2 to 7 | P |
| 8[b] | 1.00 | G | 0.52 | Mg(OH)$_2$ | 2.28 | 2 | — |

[a] – Run 6 hours.

[b] – Run at 200°C; pressure drop from 35 to 5 psig (240 to 34 kPa) during run.

## TABLE 2

| | H$_2$O Added | Carbon | Base | | Product Analysis | | | |
|---|---|---|---|---|---|---|---|---|
| Example | g | Type | g | Type | g | Carboxylic Acid, C$_n$ | Alkane | Aromatic |
| 9 | 1.00 | G | 0.52 | KOH | 0.65 | 2 to 7 | 13 to 15 | D |
| 10 | 1.00 | G | 0.52 | KOH | 0.38 | 2 to 7 | 13 to 15 | D |
| 11 | 1.00 | G | 0.52 | NaOH | 2.24 | 2 to 7 | 12 to 15 | — |

TABLE 3

| | Feed | | | | | Product Analysis | |
| | $H_2O$ Added | Carbon | | Base | | Carbox-ylic | |
| Example | g | Type | g | Type | g | Acid, $C_n$ | Alkane |
| --- | --- | --- | --- | --- | --- | --- | --- |
| 12 | 1.00 | G | 0.52 | $K_2CO_3$ | 2.28 | 2 to 4 | 12 to 17 |
| 13 | 1.00 | G | 0.52 | $Na_2CO_3$ | 2.28 | 2, 3 | 11 to 17 |
| 14 | 1.00 | G | 0.52 | $CaCO_3$ | 2.28 | 2 | 12 to 17 |
| 15[a] | 1.00 | G | 0.52 | $MgCO_3$ | 2.28 | 2, 3 | — |

[a] — Run at 200°C; pressure was 200 psig (1.4 MPa) during run.

EXAMPLES 16 to 34

These Examples, summarized in Table 4, illustrate the process of this invention employing various carbon sources and reaction conditions. The products shown for Example 16 represent the qualitative sum of five identical runs. Likewise, Examples 17 and 18 represent the sum of five identical runs with the products worked up as for Example 16. Before the run started, 0.06 g of a nickel catalyst was added which consisted of 60 to 62% Ni on kieselguhr with $125m^2/g$ surface area (Aldrich Chemical Co.). In addition, 2000 psig (13.9 MPa) of hydrogen was charged to the shaker bomb. Less than 5% of the hydrogen was consumed during the one hour run.

It is apparent from these Examples that when the carbonaceous source contains bound hydrogen and oxygen a significant portion of the carbonaceous source is converted to $C_1$ to $C_7$ alkanes, $C_2$ to $C_7$ alkenes, $C_5$ to $C_7$ dienes and aromatics as well as the usual carboxylic acids.

In each of Examples 24 to 28, the molar ratio of metal oxide to potassium hydroxide was 0.27.

## TABLE 4

| | | Feed | | | | | | Gas Recovered |
| | H$_2$O | Carbon | | KOH | Gas Charged | | | |
| Example | g | Type | g | g | Type | P-psig | moles | moles |
|---|---|---|---|---|---|---|---|---|
| 16 | 0.40 | DEX | 0.88 | 3.26 | | Vac | 0.0097 | |
| 17 | 0.40 | DEX | 0.76 | 3.32 | H$_2$ | 2000 (13.9MPa) | 0.0427 | 0.0376 |
| 18 | 0.40 | DEX | 0.78 | 3.39 | H$_2$ | 2000 (13.9MPa) | 0.0427 | 0.0414 |
| 19 | 0 | ATL | 0.52 | 2.24 | | Vac | | 0.0049 |
| 20 | 1.00 | ATL | 0.52 | 2.24 | | Vac | | 0.0025 |
| 21 | 1.00 | ANT | 0.52 | 2.24 | | Vac | | |
| 22 | 1.00 | CWK | 0.52 | 2.24 | | Vac | | 0.0025 |
| 23 | 1.00 | WSB | 0.52 | 2.24 | | Vac | | 0.0032 |
| 24 | 1.60 | MRW | 0.52 | 2.24 | | Vac | | 0.0014 |
| 25 | 1.60 | MRW | 0.52 | 2.24 | | Vac | | 0.0013 |
| 26 | 1.60 | G | 0.52 | 2.24 | | Vac | | Vac |
| 27 | 1.60 | G | 0.52 | 2.24 | | Vac | | Vac |
| 28 | 1.60 | G | 0.52 | 2.24 | | Vac | | Vac |
| 29 | 2.13 | G | 1.04 | 4.47 | H$_2$ | 2000 (13.9MPa) | 0.0283 | 0.0215 |
| 30 | 1.60 | G | 0.52 | 2.24 | N$_2$ | 1000 (7.0MPa) | 0.0201 | 0.0147 |
| 31 | 1.60 | G | 0.52 | 2.24 | N$_2$ | 500 (3.5MPa) | 0.0102 | 0.0052 |
| 32 | 1.60 | G | 0.52 | 2.24 | N$_2$ | 200 (1.4MPa) | 0.0043 | 0.0018 |
| 33 | 1.60 | G | 0.52 | 2.24 | N$_2$ | 100 (690kPa) | 0.0023 | 0.0011 |
| 34 | 1.60 | G | 0.52 | 2.24 | N$_2$ | 50 (345kPa) | 0.0013 | 0.0007 |

TABLE 4 (continued)

## Product Analyses

| Example | n in C$_n$ | | | | | Notes/Other Products |
| | Acid | Alcohol | Alkane | Alkene | Aromatic | |
|---|---|---|---|---|---|---|
| 16[a] | 2 to 6 | | | | | |
| 17[a] | 2 to 4 | 3 | | | | Added 0.06 Ni catalyst. Ran 1 hr at 200°C. Other products - butyrolactone, CH$_2$=CHCH$_2$OCH$_2$CH$_2$OH |
| 18[a] | 2 to 5 | | | | PC | Added 0.06 Ni catalyst. Ran 1 hr. |
| 19[b] | | | 1 to 7 | 2 to 7 | BTX | Plus C$_5$ to C$_7$ dienes. |
| 20[b] | | | 1 to 7 | 2 to 7 | BTX | Plus C$_5$ to C$_7$ dienes. |
| 21[b] | | | 1 to 7 | 2 to 7 | BTX | Plus C$_5$ to C$_7$ dienes. |
| 22[b] | | | 1 to 7 | 2 to 7 | BTX | Plus C$_5$ to C$_7$ dienes. |
| 23 | 2 to 6 | | 1 to 7 | 2 to 7 | BTXPC | Plus C$_9$H$_{12}$, indans, naphthalenes, and C$_5$ to C$_7$ dienes. |
| 24 | 2,3,5 | | 1,2,3,5,6 | 3 to 6 | BTXPC | Pressure was 1500 to 1700 psi (10.4 to 11.8MPa) through run. 1.10g Al$_2$O$_3$ added to feed. Plus cyclohexane, cyclopentane, methylcyclopentene, tetrahydrofuran. |
| 25 | 2,3,5 | | 1,2,3,5,6 | 3 to 6 | BTXPC | 0.75g of B$_2$O$_3$ added. Pressure was 1500 to 1700 psi (10.4 to 11.8MPa) during run. |
| 26 | | 1,3,4 | | 4 | P | 1.10g Al$_2$O$_3$ added. Plus tetrahydrofuran, C$_6$H$_9$OCH$_3$, 1-methoxy-2-butene, diethyl phthalate. |
| 27 | | 1,4,5 | | 4 | B | 0.75g B$_2$O$_3$ added. Plus tetrahydrofuran. |
| 28 | | 1 | | 4 | | 0.94g MnO$_2$ added. |
| 29 | 2 to 6 | | | | | 0.104g Ni catalyst added. |
| 30[b] | | | | | BT | Plus pyridine, NH$_3$ and amino acids. |
| 31[b] | | | | | B | Plus pyridine, NH$_3$ and amino acids. |
| 32[c] | | | 2 | | BT | Plus NH$_3$. |
| 33[c] | | | 2 | 3 | BT | Plus NH$_3$. |
| 34[c] | | | 2 | 3 | BT | |

a – No gas phase analysis was carried out.

b – Pyridine was detected by GC/MS analysis before acidification of the reaction mixture.

c – No liquid phase analysis was carried out.

17

The degree of conversion of carbonaceous sources was determined for several Examples as shown in Table 5. The conversion was determined as follows. The acidified liquid and solid contents from a shaker bomb were filtered and the filter paper was thoroughly washed. After drying, the weight of the residue on the filter paper was measured and the conversion calculated. Since the filter paper residue can include aqueous insoluble products as well as unconverted carbonaceous material, the actual conversion may be higher than that shown in Table 5.

TABLE 5

Degree of Conversion of

Carbon-Source Material

| Example | Carbonaceous Material | Base | Conversion In Percent |
|---------|-----------------------|------|-----------------------|
| 5 | Graphite | KOH | 32 |
| * | Graphite | $Na_2CO_3$ | 61 |
| 24 | MRW | KOH | 63 |
| 25 | MRW | KOH | 84 |

*Duplicate of Example No. 13

The following Table summarizes
calculated reactant ratios (vi), (vii) and (viii).

### TABLE 6

### Calculated Reactant Ratios

| Example No. | $\dfrac{M^{+n}}{C}$ | $\dfrac{M^{+n}}{H_2O}$ | $\dfrac{C}{H_2O}$ |
|---|---|---|---|
| 1 | 0.78 | 0.46 | 0.59 |
| 2 | 0.78 | 0.26 | 0.33 |
| 3 | 0.78 | 0.14 | 0.18 |
| 4 | 0.78 | 0.44 | 0.56 |
| 5 | 0.35 | 0.24 | 0.68 |
| 6 | 0.06 | 0.05 | 0.76 |
| 7 | 0.71 | 0.56 | 0.79 |
| 8 | 0.90 | 0.71 | 0.79 |
| 9 | 0.23 | 0.16 | 0.71 |
| 10 | 0.13 | 0.10 | 0.71 |
| 11 | 1.29 | 1.02 | 0.79 |
| 12 | 0.76 | 0.60 | 0.79 |
| 13 | 0.99 | 0.78 | 0.79 |
| 14 | 0.53 | 0.41 | 0.79 |
| 15 | 0.58 | 0.34 | 0.58 |
| 16 | 1.63 | 1.00 | 0.61 |
| 17 | 1.93 | 1.00 | 0.52 |
| 18 | 1.92 | 1.02 | 0.53 |
| 19 | 1.37 | – | – |
| 20 | 1.37 | 0.46 | 0.33 |
| 21 | 0.78 | 0.46 | 0.59 |
| 22 | 1.09 | 0.46 | 0.42 |
| 23 | 1.15 | 0.46 | 0.40 |
| 24 | 1.42 | 0.32 | 0.22 |
| 25 | 1.42 | 0.32 | 0.22 |
| 26 | 0.78 | 0.32 | 0.40 |
| 27 | 0.78 | 0.32 | 0.40 |
| 28 | 0.78 | 0.32 | 0.40 |
| 29 | 0.78 | 0.58 | 0.56 |
| 30 | 0.78 | 0.32 | 0.40 |
| 31 | 0.78 | 0.32 | 0.40 |
| 32 | 0.78 | 0.32 | 0.40 |
| 33 | 0.78 | 0.32 | 0.40 |
| 34 | 0.78 | 0.32 | 0.40 |

CLAIMS:·

1.    A method for making organic compounds which contain carbon, hydrogen, and at least one element selected from the group consisting of oxygen, nitrogen and sulfur, characterized by contacting these reactants:

       (i)    a carbon-source,

      (ii)   water,

    (iii)  base and, optionally,

    (iv)   a heteroatom-source,

at temperatures of about 200°C to 450°C, the method characterized further in that:

    (v)    the base is selected from alkali and alkaline earth metal hydroxides and carbonates having a cation, $M^{+n}$, wherein n is 1 or 2,

    (vi)   the molar ratio of $M^{+n}/C$ is at least about 0.06/n,

    (vii)  the molar ratio of $M^{+n}/water$ is at least about 0.05/n, and

    (viii) the molar ratio of carbon in the carbon-source/water is at least about 0.2.

2.    A method according to Claim 1 wherein the carbon source is selected from the group coals, lignites, and carbohydrates.

3.    A method according to Claim 1/wherein   or Claim 2
the base is selected from the group potassium hydroxide, sodium hydroxide, calcium hydroxide, magnesium hydroxide, potassium carbonate, sodium carbonate, calcium carbonate and magnesium carbonate, lithium hydroxide and lithium carbonate.

}182

4. A method according to any one of Claims 1 to 3 employing a heteroatom-source.

5. A method according to Claim 4 wherein the heteroatom-source is selected from the group nitrogen, $Al_2O_3$ and $B_2O_3$.

6. A method according to Claim 5 wherein the heteroatom-source is nitrogen comprising contacting the reactants at reaction pressures above about 35 atmospheres.

7. A method according to any one of Claims 1 to 6 wherein the molar ratio of $M^{+n}/C$ is about $0.06/n$ to $2/n$, the molar ratio of $M^{+n}/water$ is about $0.05/n$ to $2/n$, and the molar ratio of carbon in the carbon-source/water is about 0.2 to 5.

8. A method according to any one of Claims 1 to 7 wherein $MnO_2$ is added to the reaction mixture.